# EUROPÄISCHE PATENTSCHRIFT

(11) **EP 0 895 787 B1**
(45) Veröffentlichungstag und Bekanntmachung des Hinweises auf die Patenterteilung: **02.01.2004**
(21) Anmeldenummer: 98114600.4
(22) Anmeldetag: 04.08.1998
(51) Int. Cl.: A61M 1/16, A61M 5/168

(54) **Verfahren zur Überwachung eines Gefässzuganges während einer Dialysebehandlung und Vorrichtung zur Dialysebehandlung mit einer Einrichtung zur Überwachung eines Gefässzuganges**
Method for monitoring a blood vessel access during a dialysis treatment and apparatus for dialysis treatment with a device for monitoring a blood vessel access
Procédé de surveillance d'un accès à un vaisseau sanguin pendant un traitement de dialyse et appareil pour traitement de dialyse avec un dispositif de surveillance d'un accès à un vaisseau sanguin

(30) Priorität: 06.08.1997 DE 19734002
(43) Veröffentlichungstag der Anmeldung: 10.02.1999
(73) Patentinhaber: Fresenius Medical Care Deutschland GmbH, 61352 Bad Homburg v.d.H. (DE)
(72) Erfinder: Goldau, Rainer, 97440 Schraudenbach (DE)
(74) Vertreter: Luderschmidt, Schüler & Partner GbR

(56) Entgegenhaltungen:
- EP-A- 0 332 330
- EP-A- 0 611 228
- WO-A-97/10013
- DE-A- 4 239 937
- US-A- 4 501 583

## Beschreibung

Die Erfindung betrifft ein Verfahren zur Überwachung eines Gefäßzuganges während einer Dialysebehandlung und eine Vorrichtung zur Dialysebehandlung mit einer Einrichtung zur Überwachung eines Gefäßzuganges.

Zur Entfernung von harnpflichtigen Substanzen wird das Blut eines Patienten in einem extrakorporalen Kreislauf durch eine Kammer eines von einer semipermeablen Membran in zwei Kammern unterteilten Dialysators geleitet, während die andere Kammer von einer Dialysierflüssigkeit durchströmt wird. Als Zugang zum Gefäßsystem wird häufig eine arteriovenöse Fistel angelegt, es ist aber auch der Einsatz eines Implantats möglich.

Das Blut wird dem Patienten über eine arterielle Nadel entnommen, die an die arterielle Blutleitung des extrakorporalen Kreislaufs angeschlossen ist und wird dem Patienten über eine venöse Nadel, die an die venöse Blutleitung angeschlossen ist wieder zugeführt.

Aus der DE-A-28 38 414 ist eine Dialysevorrichtung bekannt, die eine volumetrische Bilanziereinrichtung aufweist. Die Bilanziereinrichtung besteht aus zwei durch jeweils ein verschiebbares Element unterteilten Kammern, die jeweils eine Züführleitung für frische und eine mit einem Auslaß verbundene Abführleitung für verbrauchte Dialysierflüssigkeit aufweisen. In den Zuführ- und Abführleitungen sind Absperrventile angeordnet, die von einer Steuereinheit angesteuert und geschaltet werden. Zum Fördern der verbrauchten Dialysierflüssigkeit ist im Dialysierflüssigkeitsweg zwischen Dialysator und Bilanziereinrichtung eine Pumpe angeordnet.

Für die Sicherheit des Patienten während der Dialysebehandlung ist eine Überwachung des Gefäßzuganges von entscheidender Bedeutung. So ist das Herausrutschen der venösen Nadel beispielsweise mit einem größeren Blutverlust für den Patienten verbunden, wenn dieser Fehler nicht sofort bemerkt wird.

Aus dem Bereich der Infusionstechnik sind Schutzsysteme zur Überwachung des Gefäßzuganges bekannt. Die EP-A-0 328 163 beschreibt eine Infusionsvorrichtung mit einem Druckwandler in der Infusionsleitung, mit dem sich die Herzschläge des Patienten in der Infusionsleitung nachweisen lassen, sofern die Nadel zu dem Gefäßsystem einen Zugang hat. Ein fehlerhafter Gefäßzugang wird also dadurch erkannt, daß die Herzschläge nicht mehr als Druckpulse in der Infusionsleitung gemessen werden.

Aus der EP-A-0 328 162 ist eine Infusionsvorrichtung bekannt, bei der die von der Infusionspumpe in der Infusionsleitung erzeugten Druckpulse überwacht werden. Das Herausrutschen einer Nadel wird durch eine Veränderung der Form der Druckpulse erkannt.

Eine bekannte Dialysevorrichtung mit einer Einrichtung zur Überwachung eines Gefäßzuganges weist einen in der venösen Blutleitung angeordneten Druckwandler auf. Mit dem Druckwandler wird ein Druckabfall erkannt, der beim Herausrutschen der Nadel auftritt. Eine Studie über die venöse Drucküberwachung bei Dialysevorrichtungen hat aber gezeigt, daß die Überwachung des venösen Rücklaufdrucks als Schutzsystem gegenüber Blutverlust in die Umgebung beim Herausrutschen der Nadel versagen kann.

Die WO 97/10013 beschreibt eine Dialysevorrichtung mit einem Überwachungssystem, das die in der arteriellen Blutleitung durch die Blutpumpe erzeugten Druckpulse in der venösen Blutleitung überwacht. Dieses Schutzsystem hat den Nachteil, dass die Druckpulse im extrakorporalen Blutkreislauf erzeugt werden. Hierzu sind auf der Blutseite entsprechende Mittel vorzusehen.

Die EP 0 611 228 A2 beschreibt eine extrakorporale Blutbehandlungsvorrichtung, bei der der Druck in der Blutleitung überwacht wird. Wenn der Druck unter einen vorbestimmten Wert fällt, löst die Vorrichtung einen Alarm aus. Aus der DE 42 39 937 A1 ist bekannt, dass die Bilanziereinrichtung im Dialysierflüssigkeitsweg einer Hämodialysevorrichtung Druckimpulse erzeugen kann. Zur Überprüfung der Funktionsfähigkeit einer Teileinrichtung der Dialysevorrichtung wird vorgeschlagen, während der Dialysebehandlung in periodischen Zeitabständen den Dialysator vom Dialysierflüssigkeitskreislauf abzutrennen und einen Druckhaltetest im Diaylsierflüssigkeitskreislauf durchzuführen.

Der Erfindung liegt die Aufgabe zugrunde, ein Verfahren zur Überwachung eines Gefäßzuganges während einer Dialysebehandlung zu schaffen, das die Erkennung eines fehlerhaften Gefäßzugangs mit hoher Zuverlässigkeit erlaubt, ohne dass umfangreiche Veränderungen an der Dialysevorrichtung erforderlich sind. Diese Aufgabe wird erfindungsgemäß mit den Merkmalen des Patentanspruchs 1 gelöst.

Eine weitere Aufgabe der Erfindung liegt darin, eine Vorrichtung zur Dialysebehandlung mit einer Einrichtung zur Überwachung eines Gefäßzuganges zu schaffen, die einen fehlerhaften Gefäßzugang mit hoher Zuverlässigkeit erkennt und mit verhältnismäßig einfachen technischen Mitteln realisiert werden kann. Die Lösung dieser Aufgabe erfolgt erfindungsgemäß mit den Merkmalen des Patentanspruchs 8.

Bei dem erfindungsgemäßen Verfahren ist eine Druckpulsanregung im extrakorporalen Kreislauf nicht notwendig. Insofern kann das Verfahren auf den bekannten Dialysevorrichtungen unter Ausnutzung bereits vorhandener Einrichtungen implementiert werden. Die Druckpulse werden nicht auf der Blutseite, sondern auf der Dialysatseite des Dialysators erzeugt. Die im Dialysierflüssigkeitsweg erzeugten Druckpulse werden dann im extrakorporalen Kreislauf überwacht und bei einer charakteristischen Veränderung der Druckpulse im extrakorporalen Kreislauf wird auf einen fehlerhaften Gefäßzugang, d. h. das Herausrutschen der Nadel geschlossen.

In Versuchen hat sich in überraschender Weise gezeigt, dass auf der Dialysatseite erzeugte Druckpulse auch auf der Blutseite nachgewiesen werden können. Derartige Druckpulse können prinzipiell von jedem Ventil oder jeder Pumpe erzeugt werden, die auf der Dialysatseite angeordnet sind. So lassen sich die beim Umschalten der Bilanzierkammern einer Bilanziereinrichtung auf der Dialysatseite erzeugten Druckpulse auch auf der Blutseite nachweisen. Zur (negativen) Druckanregung wird bei dem erfindungsgemäßen Verfahren vorteilhafterweise von der in den Dialysevorrichtungen ohnehin vorhandenen Bilanziereinrichtung Gebrauch gemacht, die zur Bilanzierung von frischer und verbrauchter Dialysierflüssigkeit in den Dialysierflüssigkeitsweg geschaltet ist. Somit sind im extrakorporalen Kreislauf keine Mittel zur Druckerzeugung mehr erforderlich. Die von der Bilanziereinrichtung erzeugten Druckpulse können in der arteriellen oder venösen Blutleitung mit den Drucksensoren überwacht werden, die ebenfalls in den Dialysevorrichtungen ohnehin vorhanden sind. Alternativ können zur Druckpulserzeugung auch die Ultrafiltrationspumpe, die Substituatpumpe oder weitere Ventile verwendet werden, die im Dialysierflüssigkeitsweg stromauf bzw. stromab der Dialsierflüssigkeitskammer des Dialysators vorgesehen sind.

Die Druckpulse können in der arteriellen Blutleitung sogar stromauf der arteriellen Blutpumpe gemessen werden. Zweckmäßigerweise erfolgt die Druckpulsüberwachung aber in der venösen Blutleitung, um das Herausrutschen der venösen Nadel zu erkennen.

Die Druckpulse werden von der Bilanziereinrichtung immer dann erzeugt, wenn die Bilanzkammerhälften durch die Ansteuerung der in den Bilanzkammerzuführund -abführleitungen angeordneten Sperrorgane umgeschaltet werden. Da die Steuerzeiten der Ventile bekannt sind, lassen sich die von der Bilanziereinrichtung erzeugten Druckpulse einfach aus anderen asynchronen Signalen herausfiltern.

Das Herausrutschen der Nadel wird durch eine Veränderung einer charakteristischen Eigenschaft der im extrakorporalen Kreislauf überwachten Druckpulse erkannt. Wenn die Nadel herausrutscht, zeigen die Druckpulse ein deutlich ausgeprägtes Ausschwingverhalten, das auf die Reflexion der Druckwellen am dann "freien Ende" der Blutleitung zurückzuführen ist. Das Herausrutschen der Nadel wird vorteilhafterweise dadurch erkannt, daß zu Beginn der Dialysebehandlung der periodische Druckverlauf im extrakorporalen Kreislauf bei ordnungsgemäßem Gefäßzugang, d.h. gesteckter Nadel, gemessen und in einem Speicher abgelegt wird. Während der Dialysebehandlung wird der Druckverlauf im extrakorporalen Kreislauf dann fortlaufend gemessen und zur Überwachung des Ausschwingverhaltens mit dem gespeicherten Druckverlauf verglichen. Wenn eine bestimmte Signalabweichung vorliegt, die auf das Auftreten des charakteristischen Ausschwingverhaltens zurückzuführen ist, wird auf einen fehlerhaften Gefäßzugang geschlossen. Es ist aber auch möglich, die Signalabweichung zwischen dem Maximum, das nach jedem Druckpuls auftritt, und dem Minimum, das dem Maximum folgt, zu bestimmen und mit einem vorgegebenen Schwellwert zu vergleichen, wobei auf einen fehlerhaften Gefäßzugang dann geschlossen wird, wenn die Signalabweichung größer als der Schwellwert ist.

Für den Fall, daß der Gefäßzugang fehlerhaft ist, wird vorzugsweise ein Alarm gegeben. Darüber hinaus kann der Blutfluß im extrakorporalen Kreislauf unterbrechen werden, um einen Blutverlust zu vermeiden.

Das erfindungsgemäße Verfahren, das auf der Messung von im Dialysierflüssigkeitsweg erzeugten Druckpulsen im extrakorporalen Blutkreislauf beruht, kann auch mit anderen Verfahren zur Erkennung eines fehlerfaften Gefäßzuganges, beispielsweise der Überwachung eines Druckabfalls im extrakorporalen Kreislauf, kombiniert werden. Dadurch wird die Sicherheit des Überwachungssystems noch weiter erhöht.

Im folgenden wird ein Ausführungsbeispiel der Erfindung unter Bezugnahme auf die Zeichnungen näher erläutert.

Es zeigen:
- Figur 1: eine vereinfachte schematische Darstellung einer bevorzugten Ausführungsform einer Hämodiafiltrationsvorrichtung mit einer Einrichtung zur Überwachung eines Gefäßzuganges und
- Figur 2: den zeitlichen Verlauf des Blutdruckes in der venösen Blutleitung bei gesteckter und herausgezogener Nadel.

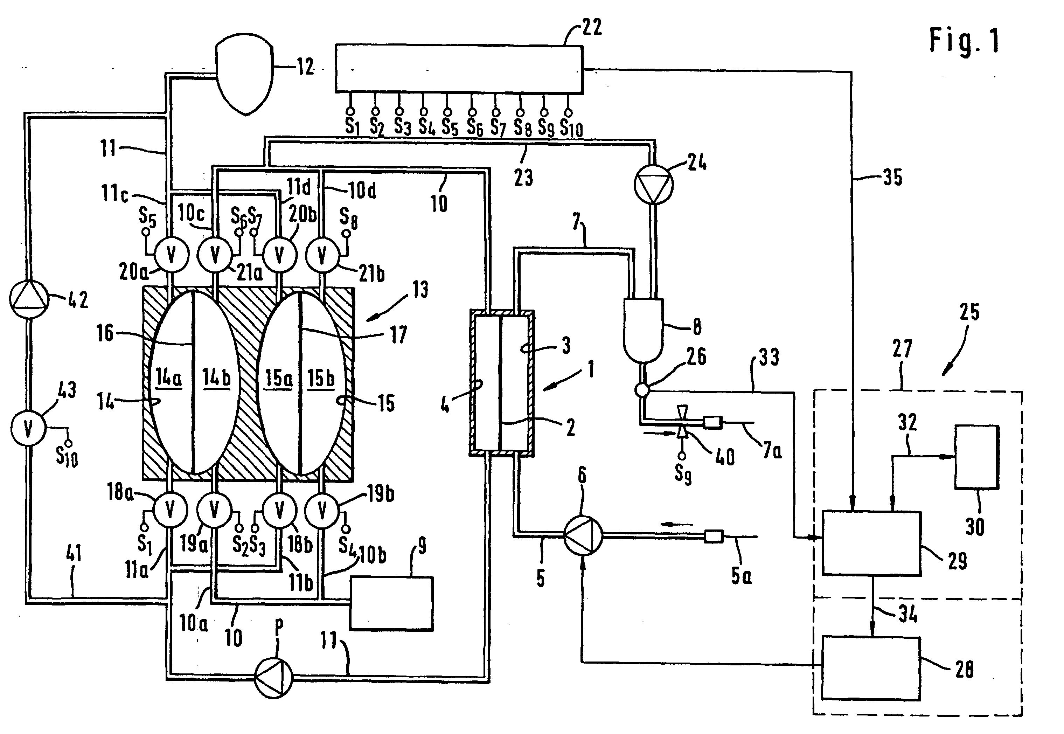

Figur 1 zeigt eine bevorzugte Ausführungsform einer Hämodiafiltrationsvorrichtung in vereinfachter schematischer Darstellung. Die Hämodiafiltrationsvorrichtung weist einen Dialysator 1 auf, der durch eine semipermeable Membran 2 in eine Blutkammer 3 und eine Dialysierflüssigkeitskammer 4 unterteilt ist. An dem Einlaß der Blutkammer 3 ist eine arterielle Blutleitung 5 angeschlossen, in die eine Blutpumpe 6 geschaltet ist. Stromab der Blutkammer 3 führt eine venöse Blutleitung 7 von dem Auslaß der Blutkammer zu dem Patienten. In die venöse Blutleitung 7 ist eine Tropfkammer 8 geschaltet. An die Enden der arteriellen und venösen Blutleitung 5, 7 sind Nadeln 5a, 7a angeschlossen, die in den arteriellen bzw. venösen Teil der Fistel des Patienten gestochen werden. In die venöse Blutleitung 7 ist eine venöse Schlauchklemme 40 geschaltet, die elektromagnetisch betätigbar ist.

In einer Dialysierflüssigkeitsquelle 9 wird frische Dialysierflüssigkeit bereitgestellt. Von der Dialysierflüssigkeitsquelle 9 führt eine Dialysierflüssigkeitszuführleitung 10 zu dem Eingang der Dialysierflüssigkeitskammer 4 des Dialysators 1, während eine Dialysierflüssigkeitsabführleitung 11 von dem Ausgang der Dialysierflüssigkeitskammer zu einem Abfluß 12 führt. Zur Bilanzierung der Dialysierflüssigkeit ist in die Dialysierflüssigkeitszuführ- und -abführleitung 10, 11 eine Bilanziereinrichtung 13 geschaltet. Eine Dialysierflüssigkeitspumpe P ist stromab des Dialysators 4 in die Dialysierflüssigkeitsabführleitung 11 geschaltet.

Die Bilanziereinrichtung 13 weist zwei Bilanzkammern 14, 15 mit dem gleichen Füllvolumen auf, die jeweils durch eine bewegliche Trennwand 16, 17, z.B. in Form einer flexiblen Membran, in eine erste Bilanzkammerhälfte 14a bzw. 15a und eine zweite Bilanzkammerhälfte 14b bzw. 15b unterteilt sind.

Der zu der Bilanziereinrichtung 13 führende Teil der Dialysierflüssigkeitszuführleitung 10 teilt sich in zwei Leitungszweige 10a, 10b auf, von denen der Leitungszweig 10a zu dem Einlaß der zweiten Kammerhälfte 14b der ersten Bilanzkammer 14 und der andere Leitungszweig 10b zu dem Einlaß der zweiten Kammerhälfte 15b der zweiten Bilanzkammer 15 führt und der von der Bilanziereinrichtung 13 wegführende Teil der Dialysierflüssigkeitszuführleitung 10 teilt sich in zwei Leitungszweige 10c, 10d auf, von denen der eine Leitungszweig 10c mit dem Auslaß der zweiten Kammerhälfte 14b der ersten Bilanzkammer 14 und der andere Leitungszweig 10d mit dem Auslaß der zweiten Kammerhälfte 15b der zweiten Bilanzkammer 15 verbunden ist.

Der zu der Bilanziereinrichtung 13 führende Teil der Dialysierflüssigkeitsabführleitung 11 teilt sich ebenfalls in zwei Leitungsabschnitte 11a, 11b auf, von denen der eine Leitungsabschnitt 11a mit dem Einlaß der ersten Kammerhälfte 14a der ersten Bilanzkammer 14 und der andere Leitungsabschnitt 11b mit dem Einlaß der ersten Kammerhälfte 15a der zweiten Bilanzkammer 15 verbunden ist. Der Auslaß der ersten Kammerhälfte 14a der ersten Bilanzkammer 14 ist über den Leitungszweig 11c und der Auslaß der ersten Kammerhälfte 15a der zweiten Bilanzkammer 15 ist über den Leitungszweig 11d der Dialysierflüssigkeitsabführleitung 11 mit dem Abfluß 12 verbunden. In den einzelnen Leitungszweigen 10a bis 10d und 11a bis 11d sind Absperrorgane in Form von elektromagnetisch betätigbaren Ventilen 18a, 18b, 19a, 19b, 20a, 20b und 21a, 21b vorgesehen, die über Steuerleitungen S, bis S₈ an einer zentralen Steuereinheit 22 angeschlossen sind. Die venöse Schlauchklemme 40 ist über eine Steuerleitung S₉ zur Betätigung derselben an die Steuereinheit 22 angeschlossen.

Von der Dialysierflüssigkeitszuführleitung 10 zweigt stromab der Bilanziereinrichtung eine Substituatzuführleitung 23 ab, in die eine Substituatpumpe 24 geschaltet ist. Die Substituatzuführleitung 23 führt in die in der venösen Blutleitung angeordnete Tropfkammer 8 (Post-Dilution). Alternativ kann die Substituatzuführleitung aber auch an eine stromauf des Dialysators angeordnete Tropfkammer angeschlossen sein (Pre-Delution).

Von der Dialysierflüssigkeitsabführleitung zweigt stromab der Dialysierflüssigkeitspumpe P eine Ultrafiltrationsleitung ab, in die eine Ultrafiltrationspumpe 42 zum Abziehen von Ultrafiltrat geschaltet ist. Die Ultrafiltrationsleitung 41 führt zu dem Abfluß 12. Stromauf der Ultrafiltrationspumpe 42 ist in der Ultrafiltrationsleitung 41 ein Ventil 43 angeordnet, um die Ultrafiltrationsleitung unterbrechen zu können. Das Ventil 43 wird von der Steuereinheit 22 über die Steuerleitung S₁₀ angesteuert.

Die Hämodiafiltrationsvorrichtung arbeitet wie folgt.

In einem ersten Bilanziertakt werden die Ventile 18a, 19b, 21a und 20b von der zentralen Steuereinheit 22 geöffnet, wobei alle anderen Ventile geschlossen werden.

Frische Dialysierflüssigkeit strömt aus der Dialysierflüssigkeitsquelle 9 in die zweite Kammerhälfte 15b der zweiten Bilanzkammer 15, wodurch verbrauchte Dialysierflüssigkeit, die in einem vorhergehenden Takt in die erste Kammerhälfte 15a geleitet wurde, in den Abfluß 12 verworfen wird. Gleichzeitig wird mittels der Dialysierflüssigkeitspumpe P verbrauchte Dialysierflüssigkeit aus der Dialysierflüssigkeitskammer 4 des Dialysators 1 in die erste Kammerhälfte 14a der ersten Bilanzkammer 14 gefördert, wodurch zuvor in die zweite Kammerhälfte 14b geleitete frische Dialysierflüssigkeit aus der zweiten Kammerhälfte verworfen und der Dialysierflüssigkeitskammer 4 zugeführt wird.

In einem zweiten Bilanziertakt werden die Ventile 18b, 19a, 20a und 21b geöffnet, wobei alle anderen Ventile geschlossen werden. Frische Dialysierflüssigkeit strömt in die zweite Kammerhälfte 14b der ersten Bilanzkammer 14 der Bilanziereinrichtung 13, wodurch verbrauchte Dialysierflüssigkeit aus der ersten Kammerhälfte 14a in den Abfluß verworfen wird. Gleichzeitig wird verbrauchte Dialysierflüssigkeit in die erste Kammerhälfte 15a der zweiten Bilanzkammer 15 gefördert, wodurch frische Dialysierflüssigkeit aus der zweiten Kammerhälfte 15b verworfen und der Dialysierflüssigkeitskammer 4 zugeführt wird.

Bei eingeschalteter Ultrafiltrationspumpe 42 wird dem geschlossenen System Flüssigkeit entzogen.

Die Hämodiafiltrationsvorrichtung verfügt über eine Einrichtung 25 zur Überwachung des Gefäßzugangs, d. h. des korrekten Sitzes der Nadeln. Die Überwachungseinrichtung 25 umfaßt einen in der venösen Blutleitung 7 angeordneten Drucksensor 26, eine Auswerteinheit 27 und einen Alarmgeber 28.

Die Bilanziereinrichtung 13 erzeugt beim Umschalten der Ventile 18 bis 21 in der Dialysierflüssigkeitzuführ- und -abführleitung 10, 11 negative Druckpulse, die sich in der venösen Blutleitung 7 mit dem Drucksensor 26 messen lassen.

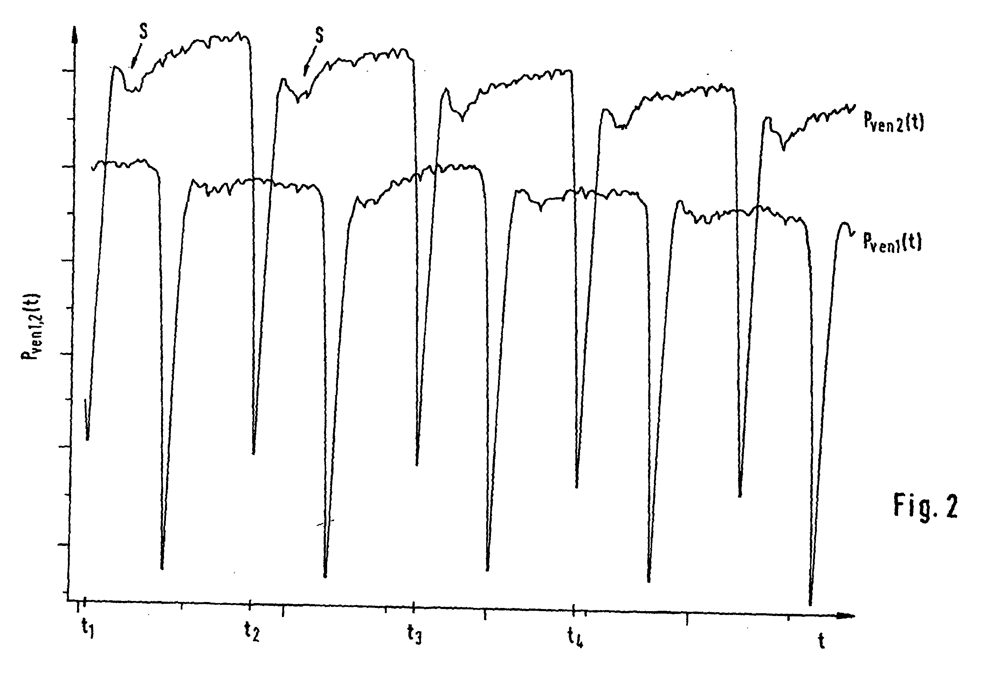

Figur 2 zeigt den zeitlichen Verlauf des von dem Drucksensor 26 erzeugten Drucksignals bei gesteckter Nadel (Pᵥₑₙ₁(t)) und bei herausgerutschter Nadel (Pᵥₑₙ₂(t)). Zu den Umschaltzeitpunkten t₁, t₂ etc. der Ventile 18 bis 21 der Bilanziereinrichtung 13 fällt der Druck in der venösen Blutleitung 7 stark ab. Wenn die venöse Nadel 7a herausgerutscht ist, zeigt sich nach dem Umschalten der Ventile eine deutlich ausgeprägte Schwingung S. Diese für einen fehlerhaften Gefäßzugang charakteristische Eigenschaft der Druckpulse im extrakorporalen Kreislauf wird von der Auswerteinheit 27 erkannt.

Die Auswerteinheit 27 weist eine Recheneinheit 29 und einen Speicher 30 auf, der über eine Datenleitung 32 mit der Recheneinheit 29 kommuniziert. Die Auswerteinheit ist über eine Signalleitung 33 mit dem Signalausgang des Drucksensors 26 und über eine Signalleitung 34 mit dem Alarmgeber 28 verbunden. Darüber hinaus empfängt die Auswerteinheit die Steuersignale der Ventile von der Steuereinheit 22 über die Datenleitung 35.

In dem Speicher 30 der Auswerteinheit 27 ist der periodische Druckverlauf in der venösen Blutleitung 7 bei ordnungsgemäßem Gefäßzugang, d.h. gesteckter Nadel, gespeichert. Dieser Druckverlauf kann von Patient zu Patient verschieden sein und auch in Abhängigkeit von dem verwendeten Schlauchsystem und dem jeweiligen Dialysator variieren. Daher wird der Druckverlauf zu Beginn der Dialysebehandlung bei ordnungsgemäßem Gefäßzugang mit dem Drucksensor 26 in der venösen Blutleitung 7 gemessen und in dem Speicher 30 abgelegt (Kalibrierung).

Während der Dialysebehandlung wird der mit dem Drucksensor 26 gemessene Druckverlauf in der Recheneinheit 29 mit dem gespeicherten Druckverlauf, der aus dem Speicher 30 ausgelesen wird, fortlaufend verglichen. Der Vergleich kann in der Recheneinheit 29 auf der Grundlage der bekannten statistischen Verfahren erfolgen, um eine Signalabweichung aufgrund der auf das Herausrutschen der Nadel zurückzuführenden Schwingung S festzustellen. So können z.B. die gemittelten Druckverläufe verglichen und bei Überschreiten einer bestimmten Standardabweichung ein fehlerhafter Gefäßzugang, d.h. das Herausrutschen der Nadel, erkannt werden. Wenn die für den fehlerhaften Gefäßzugang charakteristische Eigenschaft des Drucksignals erkannt wird, erzeugt die Auswerteinheit ein Alarmsignal, das von dem Alarmgeber 28 empfangen wird. Der Alarmgeber 28 gibt einen akustischen und/oder optischen Alarm und erzeugt ein Steuersignal für die in der arteriellen Blutleitung 5 angeordnete Blutpumpe 6. Wenn die Blutpumpe 6 das Steuersignal empfängt, wird der Blutfluß automatisch unterbrochen, so daß ein Blutverlust bei herausgerutschter Nadel nicht auftreten kann. Zusätzlich wird die venöse Klemme 40 betätigt.

Zur Erkennung eines fehlerhaften Gefäßzuganges können auch die von der Ultrafiltrationspumpe 42 oder der Substituatpumpe 24 erzeugten Druckpulse im extrakorporalen Blutkreislauf überwacht werden.

## Patentansprüche

1. Verfahren zur Überwachung eines Gefäßzuganges während einer Dialysebehandlung, bei der Blut über eine arterielle Blutleitung eines extrakorporalen Blutkreislaufs in die Blutkammer eines durch eine semipermeable Membran in die Blutkammer und eine Dialysierflüssigkeitskammer unterteilten Dialysators strömt und aus der Blutkammer über eine venöse Blutleitung des extrakorporalen Blutkreislaufs wieder zurückgeführt wird und frische Dialysierflüssigkeit über eine Dialysierflüssigkeits-Zuführleitung eines Dialysierflüssigkeitszweiges der Dialysierflüssigkeitskammer des Dialysators zugeführt wird und verbrauchte Dialysierflüssigkeit über eine Dialysierflüssigkeits-Abführleitung aus dem Dialysator abgeführt wird, wobei der Druck des Blutes im extrakorporalen Blutkreislauf überwacht und bei einer charakteristischen Veränderung des Blutdrucks auf einen fehlerhaften Gefäßzugang geschlossen wird, **dadurch gekennzeichnet, dass** im Dialysierflüssigkeitsweg Druckpulse erzeugt werden, die im extrakorporalen Blutkreislauf überwacht werden und dass bei einer charakteristischen Veränderung der Druckpulse im extrakorporalen Blutkreislauf auf einen fehlerhaften Gefäßzugang geschlossen wird.

2. Verfahren nach Anspruch 1, **dadurch gekennzeichnet, dass** im extrakorporalen Blutkreislauf die Druckpulse überwacht werden, die durch eine in die Dialysierflüssigkeits-Zuführleitung und -abführleitung geschaltete Bilanziereinrichtung erzeugt werden, die mindestens eine von einer beweglichen Trennwand in zwei Bilanzierkammerhälften unterteilte Bilanzkammer aufweist, wobei in einem ersten Bilanziertakt die eine Bilanzierkammerhälfte mit frischer Dialysierflüssigkeit unter Verwerfung von verbrauchter Dialysierflüssigkeit aus der anderen Kammerhälfte befüllt und in einem zweiten Bilanziertakt die eine Bilanzierkammerhälfte mit verbrauchter Dialysierflüssigkeit unter Verwerfung von frischer Dialysierflüssigkeit aus der anderen Bilanzierkammerhälfte befüllt wird.

3. Verfahren nach Anspruch 1 oder 2, **dadurch gekennzeichnet, dass** der Druck des Blutes im extrakorporalen Kreislauf bei ordnungsgemäßem Gefäßzugang zu Beginn der Dialysebehandlung gemessen und der periodische Druckverlauf abgespeichert wird und dass der periodische Druckverlauf im extrakorporalen Kreislauf während der Dialysebehandlung fortlaufend gemessen und mit dem abgespeicherten Druckverlauf verglichen wird, wobei bei einer charakteristischen Signalabweichung auf den fehlerhaften Gefäßzugang geschlossen wird.

4. Verfahren nach einem der Ansprüche 1 bis 3, **dadurch gekennzeichnet, dass** der Druck des Blutes in der venösen Blutleitung gemessen wird.

5. Verfahren nach einem der Ansprüche 1 bis 3, **dadurch gekennzeichnet, dass** der Druck des Blutes in der arteriellen Blutleitung gemessen wird.

6. Verfahren nach einem der Ansprüche 1 bis 5, dadurch gekenzeichnet, dass bei der Feststellung eines fehlerhaften Gefäßzugangs ein Alarm gegeben wird.

7. Verfahren nach einem der Ansprüche 1 bis 6, **dadurch gekennzeichnet, dass** bei der Feststellung eines fehlerhaften Gefäßzugangs der Blutfluss im extrakorporalen Kreislauf unterbrochen wird.

8. Vorrichtung zur Dialysebehandlung mit einer arteriellen Blutleitung (5) eines extrakorporalen Blutkreislaufs, die mit dem Einlass einer Blutkammer (3) eines durch eine semipermeable Membran (2) in die Blutkammer (13) und eine Dialysierflüssigkeitskammer (4) unterteilten Dialysators (1) verbunden ist und einer venösen Blutleitung (7), die mit dem Auslass der Blutkammer verbunden ist und einer Dialysierflüssigkeits-Zuführleitung (10) eines Dialysierflüssigkeitswegen, die mit dem Einlass der Dialysierflüssigkeitskammer verbunden ist und einer Dialysierflüssigkeits-Abführleitung (11), die mit dem Auslass der Dialysierflüssigkeitskammer verbunden ist und einer Einrichtung (25) zur Überwachung eines Gefäßzugangs während der Dialysebehandlung, die einen den Druck des Blutes im extrakorporalen Kreislauf überwachenden Drucksensor (26) und einen das Drucksignal des Drucksensors überwachende Auswerteinheit (27) aufweist, die bei einer charakteristischen Veränderung des Drucksignals auf einen fehlerhaften Gefäßzugang schließt, **dadurch gekennzeichnet, dass** im Dialysierflüssigkeitsweg (10, 11) Mittel (13) zur Erzeugung von Druckpulsen vorgesehen sind und, dass die Auswerteinheit (27) einen Speicher (30) zum Speichern eines für einen ordnungsgemäßen Gefäßzugang charakteristischen Druckverlaufs im extrakorporalen Kreislauf und eine Recheneinheit (29) aufweist, wobei die Recheneinheit (29) derart ausgebildet ist, dass in dieser der gemessene Druckverlauf im extrakorporalen Kreislauf mit dem charakteristischen Druckverlauf vergleichbar und bei einer bestimmten Signalabweichung ein fehlerhafter Gefäßzugang feststellbar ist.

9. Vorrichtung nach Anspruch 8, **dadurch gekennzeichnet, dass** die Mittel zur Erzeugung der Druckpulse eine in die Dialysierflüssigkeits-Zuführleitung (10) und die Dialysierflüssigkeits-Abführleitung (11) geschaltete Bilanziereinrichtung (13) zum Bilanzieren frischer und verbrauchter Dialysierflüssigkeit sind, die mindestens eine durch eine bewegliche Trennwand (16) in zwei Bilanzkammerhälften (14a, 14b) unterteilte Bilanzkammer (14) und in den Zuführ- und Abführleitungen der Bilanzkammerhälften angeordnete Absperrorgane (18 bis 21) aufweist.

10. Vorrichtung nach Anspruch 8 oder 9, **dadurch gekennzeichnet, dass** der Drucksensor (26) in der venösen Blutleitung (7) angeordnet ist.

11. Vorrichtung nach Anspruch 8 oder 9, **dadurch gekennzeichnet, dass** der Drucksensor (26) in der arteriellen Blutleitung (5) angeordnet ist.

12. Vorrichtung nach einem der Ansprüche 8 bis 11, **dadurch gekennzeichnet, dass** ein Alarmgeber (28) vorgesehen ist, der bei Feststellung eines fehlerhaften Gefäßzugangs einen Alarm auslöst.

13. Vorrichtung nach einem der Ansprüche 8 bis 12, **dadurch gekennzeichnet, dass** Mittel (6) zum Unterbrechen des Blutflusses im extrakorporalen Kreislauf bei der Feststellung eines fehlerhaften Gefäßzugangs vorgesehen sind.

## Claims

1. Method for monitoring a blood vessel access during a dialysis treatment, wherein blood flows via an arterial blood line of an extracorporeal blood circuit into the blood chamber of a dialyser divided by a semi-permeable membrane into the blood changer and a dialysing fluid chamber, and is fed back from the blood chamber via a venous blood line of the extracorporeal blood circuit and wherein fresh dialysing fluid is fed to the dialysing fluid chamber of the dialyser via a dialysing fluid feed line of a dialysing fluid branch and consumed dialysing fluid is discharged via a dialysing fluid discharge line from the dialyser, the pressure of the blood in the extracorporeal blood circuit being monitored and a defective vessel access being indicated by a characteristic variation in blood pressure, **characterised in that** pressure pulses are generated in the dialysing fluid path, which pulses are monitored in the extracorporeal blood circuit, and **in that** defective vessel access is indicated by a characteristic variation in the pressure pulses in the extracorporeal blood circuit.

2. Method according to Claim 1, **characterised in that** the pressure pulses which are generated by a balancing device inserted in the dialysing fluid feed line and discharge line and exhibiting at least one balance chamber divided by a mobile partition into two balancing chamber halves, are monitored in the extracorporeal blood circuit, wherein one balancing chamber half is filled in a first balancing cycle with fresh dialysing fluid, with discharge of consumed dialysing fluid from the other chamber half, and wherein one balancing chamber half is filled in a second balancing cycle with consumed dialysing fluid with discharge of fresh dialysing fluid from the other balancing chamber half.

3. Method according to Claim 1 or 2, **characterised in that** the pressure of the blood in the extracorporeal circuit is measured when the vessel access is in order at the beginning of the dialysis treatment and the periodic pressure curve is stored in a memory, and **in that** the periodic pressure curve in the extracorporeal circuit is measured continuously during the dialysis treatment and compared with the stored pressure curve, wherein a defective vessel access is indicated by a characteristic signal deviation.

4. Method according to one of Claims 1 to 3, **characterised in that** the pressure of the blood in the venous blood line is measured.

5. Method according to one of Claims 1 to 3, **characterised in that** the pressure of the blood in the arterial blood line is measured.

6. Method according to one of Claims 1 to 5, **characterised in that** an alarm is given if a defective vessel access is detected.

7. Method according to one of Claims 1 to 6, **characterised in that** the blood flow in the extracorporeal circuit is interrupted if a defective vessel access is detected.

8. Device for dialysis treatment with an arterial blood line (5) of an extracorporeal blood circuit, which line is connected to the inlet of a blood chamber (3) of a dialyser (1) divided by a semi-permeable membrane (2) into the blood chamber (13) and a dialysing fluid chamber (4), with a venous blood line (7) which is connected to the outlet of the blood chamber, with a dialysing fluid feed line (10) of a dialysing fluid path, which is connected to the inlet of the dialysing fluid chamber, with a dialysing fluid discharge line (11) which is connected to the outlet of the dialysing fluid chamber and with a device (25) for monitoring a vessel access during the dialysing treatment, which device exhibits a pressure sensor (26) monitoring the pressure of the blood in the extracorporeal circuit and an evaluating unit (27) monitoring the pressure signal of the pressure sensor, which unit indicates a defective vessel access if there is a characteristic variation in the pressure signal, **characterised in that** means (13) for generating pressure pulses are provided in the dialysing fluid path (10, 11), and **in that** the evaluating unit (27) exhibits a memory (30) for storing a pressure curve in the extracorporeal circuit characteristic of a correct vessel access and a calculating unit, wherein the calculating unit (29) is designed so that the measured pressure curve in the extracorporeal circuit can be compared in it with the characteristic pressure curve, and so that a defective vessel access can be detected in the case of a certain signal deviation.

9. Device according to Claim 8, **characterised in that** the means for generating the pressure pulses comprise a balancing device (13) inserted in the dialysing fluid feed line (10) and the dialysing fluid discharge line (11) for balancing fresh and consumed dialysing fluid, which device exhibits at least one balancing chamber (14) divided by a mobile partition (16) into two balancing chamber halves (14a, 14b), and shutoff mechanisms (18 to 21) arranged in the feed and discharge lines of the balancing chamber halves.

10. Device according to Claim 8 or 9, **characterised in that** the pressure sensor (26) is arranged in the venous blood line (7).

11. Device according to Claim 8 or 9, **characterised in that** the pressure sensor (26) is arranged in the arterial blood line (5).

12. Device according to one of Claims 8 to 11, **characterised in that** an alarm transmitter (28) is provided which triggers an alarm when a defective vessel access is detected.

13. Device according to one of Claims 8 to 12, **characterised in that** means (6) are provided for interrupting the blood flow in the extracorporeal circuit when a defective vessel access is detected.

## Revendications

1. Procédé pour surveiller un accès à un vaisseau pendant un traitement de dialyse dans lequel le sang s'écoule par l'intermédiaire d'un conduit de sang artériel d'un circuit de sang extracorporel dans la chambre de sang d'un dialyseur subdivisé par une membrane semiperméable en la chambre de sang et en une chambre de liquide de dialyse et est renvoyé de la chambre de sang par le biais d'un conduit de sang veineux du circuit de sang extracorporel et du liquide de dialyse frais est amené par le biais d'un conduit d'amenée de liquide de dialyse d'une branche de liquide de dialyse de la chambre de liquide de dialyse du dialyseur et le liquide de dialyse usé est évacué du dialyseur par le biais d'un conduit d'évacuation du liquide de dialyse, où la pression du sang dans le circuit extracorporel est surveillée et, lors d'une modification caractéristique de la pression du sang, on en déduit un accès au vaisseau défectueux, **caractérisé en ce que** des impulsions de pression qui sont surveillées dans le circuit de sang extracorporel sont produites dans la voie de liquide de dialyse et **en ce que** lors d'une modification caractéristique des impulsions de pression dans le circuit de sang extracorporel, on en déduit un accès au vaisseau défectueux.

2. Procédé selon la revendication 1, **caractérisé en ce que** les impulsions de pression sont surveillées dans le circuit de sang extracorporel, impulsions de pression qui sont produites par un dispositif d'établissement de bilan branché dans le conduit d'amenée de liquide de dialyse et dans le conduit d'évacuation de liquide de dialyse, qui comporte au moins une chambre de bilan subdivisée par une paroi de séparation mobile en deux moitiés de chambre d'établissement de bilan, où dans une première période d'établissement de bilan, une moitié de chambre d'établissement de bilan est remplie de liquide de dialyse frais avec élimination de liquide de dialyse usé provenant de l'autre moitié de chambre et dans une seconde période d'établissement de bilan une moitié de chambre d'établissement de bilan est remplie de liquide de dialyse usé avec élimination du liquide de dialyse frais provenant de l'autre moitié de chambre d'établissement de bilan.

3. Procédé selon la revendication 1 ou 2, **caractérisé en ce que** la pression du sang dans le circuit extracorporel dans le cas d'un accès au vaisseau correct est mesurée au début du traitement de dialyse et l'évolution périodique de la pression est mémorisée et **en ce que** l'évolution périodique de la pression est mesurée en continu dans le circuit extracorporel pendant le traitement de dialyse et est comparé avec l'évolution de pression mémorisée, où dans le cas d'un écart de signal caractéristique, on en déduit un accès au vaisseau défectueux.

4. Procédé selon l'une des revendications 1 à 3, **caractérisé en ce que** la pression du sang dans le conduit de sang veineux est mesurée.

5. Procédé selon l'une des revendications 1 à 3, **caractérisé en ce que** la pression du sang dans le conduit de sang artériel est mesurée.

6. Procédé selon l'une des revendications 1 à 5, **caractérisé en ce qu'**une alarme est émise lors de la mise en évidence d'un accès au vaisseau défectueux.

7. Procédé selon l'une des revendications 1 à 6, **caractérisé en ce que**, lors de la mise en évidence d'un accès au vaisseau défectueux, le flux de sang dans le circuit extracorporel est interrompu.

8. Dispositif de traitement de dialyse comportant un conduit de sang artériel (5) d'un circuit de sang extracorporel, qui est relié à l'entrée d'une chambre de sang (3) d'un dialyseur (1) subdivisé par une membrane semiperméable (2) en la chambre de sang (13) et en une chambre de liquide de dialyse (4) et un conduit de sang veineux (7) qui est relié à la sortie de la chambre de sang et un conduit d'amenée de liquide de dialyse (10) d'une voie de liquide de dialyse qui est relié à l'entrée de la chambre de liquide de dialyse et un conduit d'évacuation de liquide de dialyse (11) qui est relié à la sortie de la chambre de liquide de dialyse et un dispositif (25) pour surveiller un accès à un vaisseau pendant le traitement de dialyse, qui comporte un capteur de pression (26) surveillant la pression du sang dans le circuit extracorporel et une unité d'évaluation (27) surveillant le signal de pression du capteur de pression, qui conclut à un accès au vaisseau défectueux lors d'une modification caractéristique du signal de pression, **caractérisé en ce qu'**il est prévu dans la voie de liquide de dialyse (10, 11) des moyens (13) pour produire des impulsions de pression et **en ce que** l'unité d'évaluation (27) comporte une mémoire (30) pour mémoriser une évolution de pression caractéristique d'un accès au vaisseau correct dans le circuit extracorporel et une unité de calcul (29), où l'unité de calcul (29) est agencée de telle manière que, dans celle-ci, l'évolution de pression mesurée dans le circuit extracorporel peut être comparée avec l'évolution de pression caractéristique et, lors d'un écart de signal déterminé, un accès au vaisseau défectueux peut être mis en évidence.

9. Dispositif selon la revendication 8, **caractérisé en ce que** les moyens pour produire les impulsions de pression sont un dispositif d'établissement de bilan (13) branché dans le conduit d'amenée de liquide de dialyse (10) et dans le conduit d'évacuation de liquide de dialyse (11) pour établir le bilan du liquide de dialyse frais et du liquide de dialyse usé, qui comporte au moins une chambre de bilan (14) subdivisée par une paroi de séparation mobile (16) en deux moitiés de chambre de bilan (14a, 14b) et des organes d'arrêt (18 à 21) disposés dans les conduits d'amenée et d'évacuation des moitiés de chambre de bilan.

10. Dispositif selon la revendication 8 ou 9, **caractérisé en ce que** le capteur de pression (26) est disposé dans le conduit de sang veineux (7).

11. Dispositif selon la revendication 8 ou 9, **caractérisé en ce que** le capteur de pression (26) est disposé dans le conduit de sang artériel (5).

12. Dispositif selon l'une des revendications 8 à 11, **caractérisé en ce qu'**il est prévu un dispositif d'alarme (28) qui déclenche une alarme lors de la mise en évidence d'un accès au vaisseau défectueux.

13. Dispositif selon l'une des revendications 8 à 12, **caractérisé en ce qu'**il est prévu des moyens (6) pour interrompre le flux de sang dans le circuit extracorporel lors de la mise en évidence d'un accès au vaisseau défectueux.
